(19) Europäisches Patentamt
European Patent Office
Office européen des brevets

(11) Publication number: **0 132 395**
**B1**

(12) # EUROPEAN PATENT SPECIFICATION

(45) Date of publication of patent specification: **30.09.87**

(51) Int. Cl.⁴: **C 07 D 205/08**, C 07 D 498/04 // (C07D498/04, 265:00, 205:00)

(21) Application number: **84304957.8**

(22) Date of filing: **20.07.84**

(54) **Improvements in or relating to the preparation of azetidinone sulfinic acids from cephalosporin sulfones.**

(30) Priority: **22.07.83 US 516218**

(43) Date of publication of application:
**30.01.85 Bulletin 85/05**

(45) Publication of the grant of the patent:
**30.09.87 Bulletin 87/40**

(84) Designated Contracting States:
**BE CH DE FR GB IT LI LU NL SE**

(56) References cited:
**EP-A-0 060 120**
**EP-A-0 109 816**

**JOURNAL OF THE CHEMICAL SOCIETY, PERKIN TRANSACTIONS I, no. 2, 1982, pages 595-602; C.M. PANT et al.: "Studies related to penicillins. Part 21. beta-Elimination reactions of S,S-dioxides of penicillanic esters"**

**JOURNAL OF THE CHEMICAL SOCIETY, PERKIN TRANSACTIONS I, no. 9, 1979, pages 2268-2275; C.L. BRANCH et al.: "Synthesis of some 1-oxa-1-dethiacephalosporins"**

(73) Proprietor: **ELI LILLY AND COMPANY**
**307, East McCarty Street**
**Indianapolis Indiana 46285 (US)**

(72) Inventor: **McShane, Lawrence Joseph**
**418 David Lane**
**Indianapolis Indiana 46227 (US)**

(74) Representative: **Crowther, Terence Roger et al**
**Erl Wood Manor**
**Windlesham Surrey GU20 6PH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 132 395 B1

**Description**

This invention concerns a process for converting 3-exomethylene cepham sulfones to azetidinone sulfinic acids which are useful in the synthesis of 1-oxadethiacephalosporin antibiotics.

A new class of antibiotics which have proven to be very effective against a broad spectrum of bacterial infections recently has been discovered. This new class of compounds, the 1-oxadethiacephalosporins, are cephalosporin analogs having an oxygen atom in place of the sulfur atom in the cephalosporin nucleus. These compounds are discussed by Sheehan *et al.,* in *J. Heterocyclic Chemistry, Vol. 5,* page 779 (1968); Christensen *et al.* in *J. Am. Chem. Soc. Vol. 96,* page 7582 (1974); and Narisada *et al.,* U.S. Patent No. 4,138,486.

The reported syntheses of 1-oxadethiacephalosporins have employed, inter alia, haloazetidinones such as 4-chloroazetidinones; see U.S. Patent Nos. 4,013,653, 4,234,724 and 4,159,984. These haloazetidinone starting materials generally have been prepared by reaction of a penicillin with a halogenating agent such as molecular halogen or an N-halo succinimide, U.S. Patent No. 4,159,984. Narisada *et al.,* in U.S. Patent No. 4,138,486 described the synthesis of chloroazetidinones from methylthioazetidinones which are derived from penicillins. To date, haloazetidinones have not been available from cephalosporin starting materials.

This invention provides a chemical process for converting cephalosporin sulfones to azetidinone sulfinic acids which then can be converted to haloazetidinones. These haloazetidinones then can be converted to 1-oxadethiacephalosporin antibiotics.

In particular, this invention provides a process for preparing an azetidinone sulfinic acid of Formula (I):

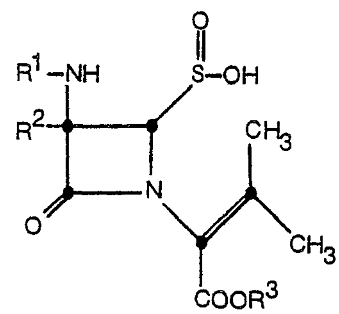

(I)

in which

$R^1$ is an acyl residue of a carboxylic acid,

$R^2$ is hydrogen, lower alkoxy or lower alkylthio, and

$R^3$ is a removable ester forming group which comprises reacting a 3-exomethylene sulfone of Formula (II):

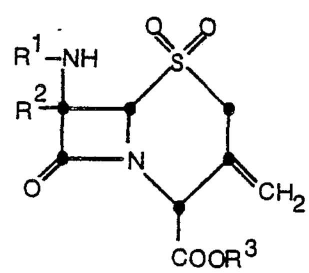

(II)

which activated zinc, magnesium, activated magnesium, or amalgamated magnesium and a protonic acid in a solvent at a temperature of about 20°C to about 100°C.

The formation of compounds of formula I by β-elimination reactions of S,S-dioxides and penecillianic esters has been described by Chandra M. Pant, *et al,* in *J. Chem. Soc., Perkin Transaction,* 5 (2) 1982, 595.

The process preferably is carried out employing a protonic acid that is, for instance, bound to an amine compound. A particularly preferred bound protonic acid is ammonium chloride.

Another preferred embodiment is to perform the process on a sulfone of the above formula in which $R^1$ is

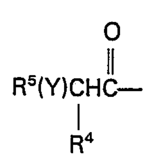 , 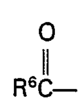

in which:

$R^4$ is hydrogen, amino, protected-amino, hydroxy, protected-hydroxy, tetrazolyl, carboxy, or protected-carboxy;

$R^5$ is hydrogen, phenyl, substituted phenyl, cyclohexadienyl, or a 5- or 6-membered monocyclic heterocyclic ring containing one or more oxygen, sulfur or nitrogen atoms in the ring, said ring being substituted with hydrogen or amino;

Y is oxygen or a direct bond; and

$R^6$ is hydrogen, phenyl, substituted phenyl, alkyl or substituted alkyl.

The process is performed conveniently using 3-exomethylene cepham sulfone substrates in which $R^1$ is

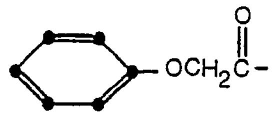 ;

a)

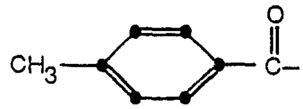 ;

b)

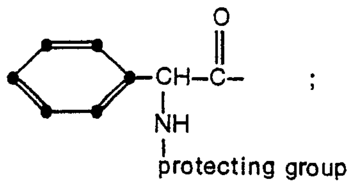 ;

c)

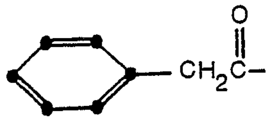 ;

d)

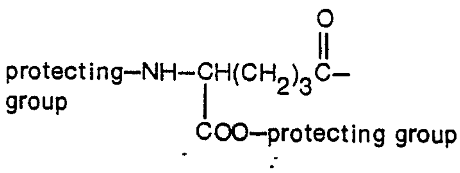

e)

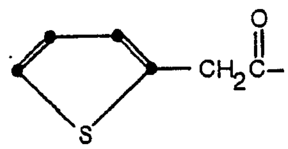 ;

f)

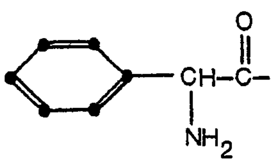 ;

g)

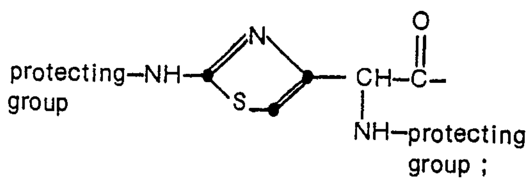

h)

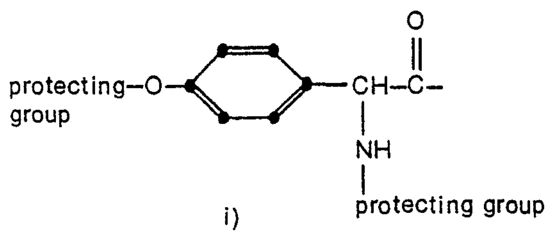

i)

A preferred metal to be employed in the process of this invention is activated zinc. A preferred reaction solvent is N,N-dimethylformamide. Solvents which are used should be unreactive under the reaction conditions used.

$R^1$ in the above formula defines an acyl residue of a carboxylic acid. Because the process of this invention operates on the ring nucleus of the cephalosporin starting material, the particular $R^1$ group is not critical to the process. Numerous and varied acyl residues of carboxylic acids are known in the cephalosporin and penicillin arts, and all such groups are contemplated as included within this invention. U.S. Patent Nos. 4,052,387 and 4,243,588, incorporated herein by reference, disclose representative and typical carboxylic acid acyl residues.

Preferred cephalosporin sulfones to be employed in the present process include those defined by the above formula in which $R^1$ is

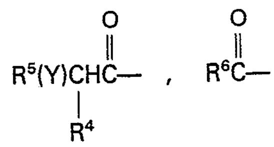

in which

Y is oxygen or a direct bond,

$R^4$ is hydrogen, amino, protected-amino, hydroxy, protected hydroxy, tetrazolyl, carboxy or protected-carboxy; and

$R^5$ is hydrogen, phenyl, substituted phenyl, cyclohexadienyl, or a 5 or 6-membered heterocyclic ring. As used herein, the terms "protected-amino," "protected-hydroxy," and "protected-carboxy" have their respective art-recognized meanings. For instance, "protected-amino" means an amino group which has been derivatized with a readily cleavable group capable of preventing unwanted side reactions of the amino group during the course of the present process, or alternatively, aids in solubilizing the amino containing substrate. Groups that are employed as protecting groups for amino, hydroxy and carboxy moieties are well-known, and many are exemplified in Chapters 2, 3 and 5 of *Protective Groups in Organic Chemistry,* McOmie, Ed., Plenum Press, New York, N.Y., (1973), and also *Protective Groups in Organic Synthesis,* Greene, Ed., John Wiley and Sons, New York, N.Y., (1981), both of which are incorporated herein by reference.

Typical amino-protecting groups include *tert*-butoxycarbonyl, benzyloxycarbonyl, 4-methoxybenzyloxycarbonyl, 4-nitrobenzyloxycarbonyl, and the 1-carbomethoxy-2-propenyl group. Commonly used hydroxy-protecting groups include acyl groups such as formyl, acetyl, chloroacetyl; arylalkyl groups such as benzyl and 4-nitrobenzyl, and alkyl groups such as methoxymethyl and *tert*-butyl. Groups routinely employed to protect carboxy groups, and which constitute what are referred to herein as "removable ester-forming groups", include alkyl groups such as methyl, *tert*-butyl, or $C_2$—$C_6$ alkanoyloxymethyl, and arylalkyl groups such as diphenylmethyl, benzyl, 4-methoxybenzyl, 4-nitrobenzyl, tri($C_1$—$C_3$ alkyl)silyl, succinimidomethyl, and related groups. When reference is made to hydroxy, amino or carboxy groups, the respective protected groups also are contemplated. All that is intended is that such groups can be substituted with conventional blocking groups used for the temporary protection of such groups against undesired side reactions, and to aid in solubilization of the compound containing such groups. Such protected groups can be converted to the corresponding free hydroxy, carboxy or amino group by conventional and well-known methods.

The term "substituted phenyl" means a phenyl group bearing one or two substituents selected from $C_1$—$C_4$ alkyl, amino, hydroxy and lower alkoxy.

Exemplary carboxylic acid acyl residues defined by

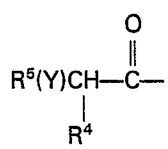

may include groups such as phenylacetyl, phenoxyacetyl, 4-cyanophenylacetyl, 4-*tert*-butoxyphenylacetyl, α-hydroxyphenylacetyl, α-aminophenylacetyl, α-*tert*-butoxycarbonylaminophenylacetyl, α-ethoxycarbonylphenylacetyl, or 4-nitrophenoxyacetyl, or protected derivatives thereof.

Another preferred $R^1$ carboxylic acid acyl residue is defined by

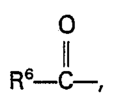

in which $R^6$ is hydrogen, alkyl, for example $C_1$—$C_6$ alkyl, phenyl, substituted phenyl or substituted alkyl. The term "substituted alkyl" means a $C_1$—$C_6$ alkyl group bearing one or more substituents selected from hydroxy, amino, carboxy and alkoxy. Exemplary

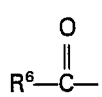

groups may include groups such as formyl, acetyl, *n*-butyryl, 5-aminopentanoyl, benzoyl, 4-aminobenzoyl, 3-hydroxybenzoyl, 4-methylbenzoyl, and 2,6-diethylbenzoyl, or protected derivatives thereof.

According to the process of this invention, a 3-exomethylene-1,1-dioxocepham (*i.e.*, a cephalosporin sulfone) is reacted with activated zinc, activated magnesium, magnesium, or amalgamated magnesium, and a protonic acid, to provide an azetidinone sulfinic acid. A preferred metal to be employed in the process

is activated zinc. Activated zinc is simply zinc metal that is substantially free of oxide coatings. Commercially available zinc metal dust generally has one or more layers of zinc oxide coating. These are removed readily by simply washing the zinc with a dilute mineral acid, for example 1N hydrochloride or 1N sulfuric acid. The activated metal formed generally is washed with a solvent that is to be employed in the process, although any common laboratory solvent can be employed. Typical solvents to be employed in the instant process may be the polar solvents such as N,N-dimethylformamide, formamide, dimethyl sulfoxide, hexamethylphosphortriamide, or N,N-dimethylacetamide. Less polar organic solvents can be employed if desired, for example alcohols such as methanol, ethanol, isopropanol, as well as ethers such as diethyl ether, methyl ethyl ether, tetrahydrofuran, and ketones such as acetone or methyl ethyl ketone. A preferred solvent for the process is N,N-dimethylformamide. If desired, more than one solvent can be employed, and a mixture of N,N-dimethylformamide and water in a volume ratio of about 80:20 is a particularly preferred solvent system.

The process of the invention is carried out in the presence of a protonic acid of which any number of common protonic acids can be utilized. Typical protonic acids commonly employed include the mineral acids such as hyrochloric acid, hydrobromic acid, sulfuric acid, nitric acid, phosphoric acid, as well as organic protonic acids such as formic acid, acetic acid, trifluoroacetic acid, chloroacetic acid, methanesulfonic acid, or benzoic acid.

If desired, the protonic acid can be employed in the form of a bound proton source, for example, as an amine acid addition salt. Typical amines commonly used to bind the protonic acid include ammonia and lower alkyl amines such as methyl amine, dimethyl amine, triethyl amine, as well as cyclic and aromatic amines such as pyrrolidine, piperizine, or pyridine. Ammonia is especially preferred and hydrochloric acid is a preferred protonic acid to be used in conjunction with ammonia (*i.e.,* ammonium chloride).

While the respective quantities of metal and protonic acid used in the process are not critical, it is preferred to use about an equimolar or excessive quantity of each to promote complete conversion of the 2-exomethylene cephalosporin sulfone. An amount of metal and acid ranging from about 1 to about 50 molar excess relative to the cepham sulfone starting material routinely is employed, although larger or smaller excesses are not detrimental and can be utilized if desired.

The reaction of the 3-exomethylene sulfone, the metal and the protonic acid generally is performed at a temperature of about 20° to about 100°C, and more typically at about 25° to about 60°C. The reaction generally is complete after about 2 to about 24 hours whene carried out within this temperature range.

The product of the present process, an azetidinone sulfinic acid, is isolated readily by routine procedures. For example, the reaction mixture can be filtered to remove any excess metal, and the filtrate can be concentrated to dryness. The product formed can be purified further, if needed, by routine methods including salt formation and crystallization. The product of the process most conveniently is not isolated, but rather is reacted further *in situ* with a halogenating agent such as N-chlorosuccinimide or N-bromosuccinimide in order to obtain a haloazetidinone of the formula

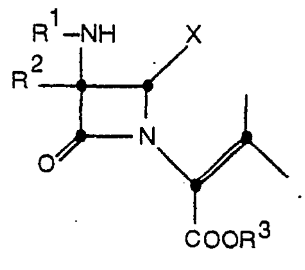

in which $R^1$, $R^2$ and $R^3$ are as defined above, and X is halo such as fluoro, chloro, bromo or iodo. Such haloazetidinones are useful in the synthesis of 1-oxadethiacephalosporin compounds which are either active as antibiotics themselves, or readily are convertible to antibiotics, for example, by removing any protecting groups present. The conversion of haloazetidinones to 1-oxadethiacephalosporins is described in U.S. Patent Nos. 4,013,653 and 4,234,724.

The process of this invention operates equally well in cepham sulfones in which the 7-acylamino side chain is in the natural or β-configuration, or in which the 7-acylamino side chain is in the α- or epi configuration. The configuration of the acylamino group is maintained throughout the process so that the azetidinone sulfinic acid product has an acylamido side chain in the same configuration as the starting material employed. Accordingly, the present process produces natural azetidinone-4-sulfinic acids of the formula:

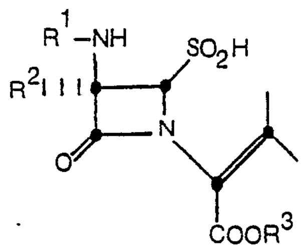

and epi-azetidinone-4-sulfinic acids of the formula:

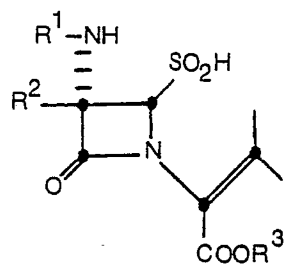

The 3-exomethylene sulfones of formula (II) used in the process of the present invention and the production thereof form the subject of our cofiled Application No. EP—A—0132394, the disclosure of which is herein incorporated by reference.

The following non-limiting examples are provided to further illustrate the invention.

Example 1

Diphenylmethyl 3-methyl-2-(2-sulfinyl-4-oxo-3-(4-methylbenzoylamino)-1-azetidinyl)-2-butenoate

A suspension of 3.18 g (6 mM) of diphenylmethyl 7-β-(4-methylphenylcarboxamido)-3-exomethylenecepham-1,1-dioxide-4-carboxylate in 35 ml of N,N-dimethylformamide and 5 ml of water was stirred at 25°C under a nitrogen blanket. Six grams of ammonium chloride was added in one portion to the reaction mixture, followed by the addition of 7.5 g of zinc metal dust that had been washed with 50 ml of 1N hydrochloric acid. The reaction mixture was stirred for twenty-four hours at 25°C, and then filtered through hyflo filter aid. The filter cake was washed with 20 ml of N,N-dimethylformamide and then with 200 ml of ethyl acetate. The filtrate was washed three times with 100 ml portions of 5% (v/v) aqueous hydrochloric acid. The organic layer was separated, washed with brine, dried, and the solvent was removed by evaporation under reduced pressure to give 3.5 g of a white foam identified as diphenylmethyl 3-methyl-2-(2-sulfinyl-4-oxo-3-(4-methylbenzoylamino)-1-azetidinyl)-2-butenoate.

IR($CHCl_3$): 1778 $cm^{-1}$

NMR ($CDCl_3$): δ2.01—2.25 (three singlets, 3H each); δ4.70 (d, 1H); δ5.60 (dd, 1H); δ6.1—7.9 (m, 16H); δ9.35 (s, 1H),

Example 2

Diphenylmethyl 3-methyl-2-(2-sodium sulfonyl-4-oxo-3-(4-methylbenzoylamino)-1-azetidinyl)-2-butenoate

To a stirred suspension of 3.18 g (6 nM) of diphenylmethyl 7-β-(4-methylphenylcarboxoamido)-3-exomethylenecepham-1,1-dioxide-4-carboxylate in 35 ml of N,N-dimethylformamide and 5 ml of water were added 6.0 g of ammonium chloride followed by addition of 7.5 g of activated zinc (activated by washing twice with dilute hydrochloric acid and twice with water). The reaction mixture was stirred at 25°C for twenty four hours under a nitrogen blanket. The reaction mixture was filtered through hyflo filter aid, and the filter cake was washed with 100 ml of ethyl acetate. The filtrate was washed with 5% aqueous hydrochloric acid and dried. The solution was stirred while a solution of 1 g (6 mM) of sodium 2-ethylhexanoate in 20 ml of ethyl acetate was added in one portion. The reaction mixture was stirred at 25°C for sixteen hours, and then the solvent was removed by evaporation under reduced pressure to provide an oil. The oil was crystallized from 50 ml of chloroform to afford 1.0 g of diphenylmethyl 3-methyl-2-(2-sodium sulfonyl-4-oxo-3-(4-methylbenzoylamino)-1-azetidinyl)-2-butenonate of the formula

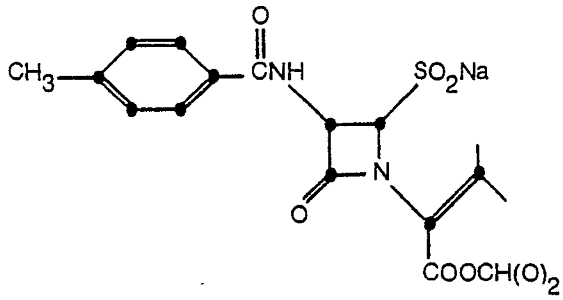

IR (KBr): 1776 $cm^{-1}$
NMR (DMSO—$d_6$): δ2.05 (s, 3H); δ2.18 (s, 3H); δ2.39 (s, 3H); δ4.56 (d, 1H); δ5.56 (dd, 1H); δ7.3—7.5 (m, 13H); δ7.70 (d, 2H); δ8.71 (d, 1H).

Example 3

Benzyl 3-methyl-2-[2-sulfinyl-4-oxo-3-methoxy-3-(phenylacetamido)-1-azetidinyl]-2-butenoate

A solution comprised of 2.5 g (5.15 mM) of benzyl 7-β-(phenylacetamido)-7-α-methoxy-3-exomethylenecepham-1,1-dioxide-4-carboxylate in 20 ml of DMF and 75 ml of ethanol was heated to 65°C and stirred while 8.25 g (154.79 mM) of ammonium chloride were added in one portion, followed by the addition of 16.83 g (257.5 mM) of activated zinc. The reaction mixture was stirred for three hours at 65°C and then cooled to about 30°C. The reaction mixture was diluted by addition of 100 ml of ethyl acetate, and the mixture was washed six times with 20 ml portions of 1N hydrochloric acid and once with brine. The organic solution was dried and concentrated to dryness to provide benzyl 3-methyl-2-[2-sulfinyl-4-oxo-3-methoxy-3-(phenylacetamido)-1-azetidinyl]-2-butenoate.

**Claims**

1. A process for preparing an azetidinone sulfinic acid of Formula (I)

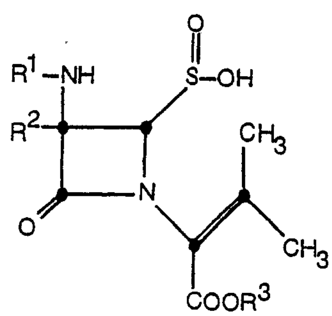

(I)

in which:
$R^1$ is an acyl residue of a carboxylic acid;
$R^2$ is hydrogen, lower alkoxy or lower alkylthio; and
$R^3$ is a removable ester forming group which comprises reacting a 3-exomethylene sulfone of Formula (II):

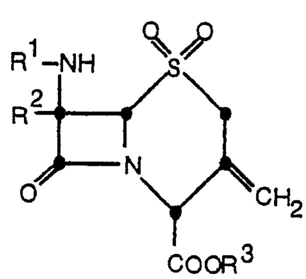

(II)

with activated zinc, magnesium, activated magnesium or amalgamated magnesium and a protonic acid in a solvent at a temperature of about 20° to about 100°C.

2. A process as claimed in claim 1 in which
$R^1$ is

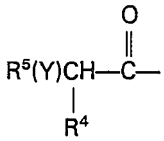 or 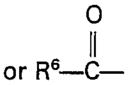

in which

$R^4$ is hydrogen, amino, protected-amino, hydroxy, protected-hydroxy, tetrazolyl, carboxy or protected-carboxy;

$R^5$ is hydrogen, phenyl, phenyl bearing one or two substituents selected from $C_1$—$C_4$ alkyl, amino, hydroxy or lower alkoxy, cyclohexadienyl, or a 5- or 6-membered monocyclic heterocyclic ring containing one or more oxygen, sulfur or nitrogen atoms in the ring, said ring being substituted with hydrogen or amino;

Y is oxygen or a direct bond; and

$R^6$ is hydrogen, phenyl, alkyl, or a $C_1$—$C_6$ alkyl group bearing one or more substituents selected from hydroxy, amino, carboxy or alkoxy.

3. A process as claimed in claim 1 or 2 in which $R^1$ is

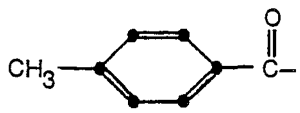 ; b)

4. A process as claimed in any one of claims 1 to 3 employing activated zinc.

5. A process as claimed in claim 4 employing N,N-dimethylformamide as reaction solvent.

6. A process for preparing an antibiotically-active oxadethiacephalosporin which comprises preparing an azetidinone sulfinic acid according to claim 1 and converting the azetidinone sulfinic acid into the antibiotically-active oxadethiacephalosporin by methods known *per se.*

**Patentansprüche**

1. Verfahren zur Herstellung einer Azetidinonsulfinsäure der Formel (I)

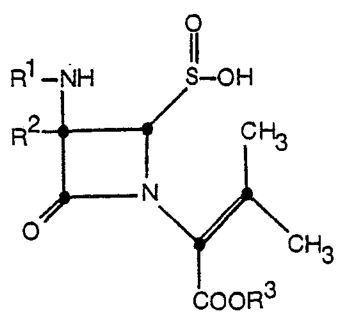

(I)

worin

$R^1$ einen Acylrest einer Carbonsäure bedeutet,

$R^2$ Wasserstoff, Niederalkoxy oder Niederalkylthio ist und

$R^3$ für eine entfernbare esterbildende Gruppe steht, dadurch gekennzeichnet, daß man ein 3-Exomethylensulfon der Formel (II)

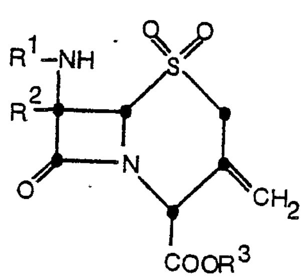

(II)

mit aktiviertem Zink, Magnesium, aktiviertem Magnesium oder amalgamiertem Magnesium und einer Protonensäure in einem Lösungsmittel bei einer Temperatur von etwa 20°C bis etwa 100°C umsetzt.

2. Verfahren nach Anspruch 1, dadurch gekennzeichnet, daß $R^1$ für

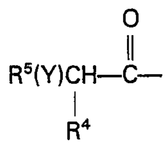 oder 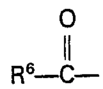

steht, worin

$R^4$ Wasserstoff, Amino, geschütztes Amino, Hydroxy, geschütztes Hydroxy, Tetrazolyl, Carboxy oder geschütztes Carboxy ist,

$R^5$ Wasserstoff, Phenyl, durch $C_1$—$C_4$-Alkyl, Amino, Hydroxy oder Niederalkoxy einfach oder zweifach substituiertes Phenyl, Cyclohexadienyl oder einen 5- oder 6-gliedrigen einkernigen heterocyclischen Ring bedeutet, der ein oder mehr Sauerstoff-, Schwefel- oder Stickstoffatome enthält und durch Wasserstoff oder Amino substituiert ist,

Y Sauerstoff oder eine direkte Bindung ist und

$R^6$ für Wasserstoff, Phenyl, durch $C_1$—$C_4$-Alkyl, Amino Hydroxy oder Niederalkoxy einfach oder zweifach substituiertes Phenyl, Alkyl oder durch Hydroxy, Amino, Carboxy oder Alkoxy einfach oder mehrfach substituiertes $C_1$—$C_6$-Alkyl steht.

3. Verfahren nach Anspruch 1 oder 2, dadurch gekennzeichnet, daß $R^1$ für

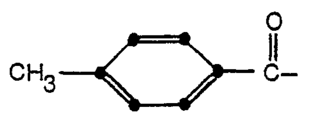 ; b)

steht.

4. Verfahren nach irgendeinem der Ansprüche 1 bis 3, dadurch gekennzeichnet, daß aktiviertes Zink verwendet wird.

5. Verfahren nach Anspruch 4, dadurch gekennzeichnet, daß N,N-Dimethylformamid als Reaktionslösungsmittel verwendet wird.

6. Verfahren zur Herstellung eines antibiotisch wirksamen Oxadethiacephalosporins, dadurch gekennzeichnet, daß man unter Anwendung an sich bekannter Methoden eine Azetidinonsulfinsäure gemäß Anspruch 1 herstellt und diese Azetidinonsulfinsäure dann zum jeweiligen antibiotisch wirksamen Oxadethiacephalosporin umwandelt.

**Revendications**

1. Procédé de préparation d'un acide azétidinone-sulfinique de formule (I):

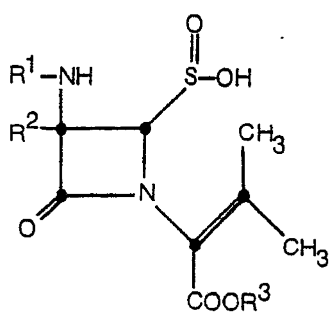 (I)

dans laquelle

$R^1$ représente un radical acyle d'un acide carboxylique;

$R^2$ représente un atome d'hydrogène, un groupe alcoxy inférieur ou un groupe alkyl inférieur-thio; et

$R^3$ représente un groupe éliminable formateur d'ester, caractérisé en ce qu'il consiste à faire réagir une 3-exométhylène-sulfone de formule (II):

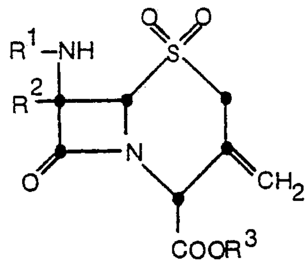

(II)

avec du zinc activé, du magnésium, du magnésium activé ou du magnésium amalgamé, et un acide protonique, dans un solvent et à une températuer compriseentre environ 20°C et environ 100°C.

2. Procédé selon la revendication 1, dans lequel $R^1$ représente

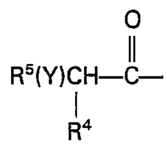 ou 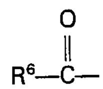

où

$R^4$ représente un atome d'hydrogène, un groupe amino, un groupe amino protégé, un groupe hydroxyle, un groupe hydroxyle protégé, un groupe tétrazolyle, un groupe carboxyle ou un groupe carboxyle protégé;

$R^5$ représente un atome d'hydrogène, un groupe phényle, un groupe phényle comportant un ou deux substituants choisis parmi un groupe alkyle en $C_1$—$C_4$, un groupe amino, un groupe hydroxyle ou un groupe alcoxy inférieur, un groupe cyclohexadiényle ou un noyau hétérocyclique monocyclique pentagonal ou hexagonal contenant un ou plusieurs atomes d'oxgène, de soufre ou d'azote, ce noyau étant substitué par un atome d'hydrogène ou par un groupe amino;

Y représente un atome d'oxygène ou une liaison directe; et

$R^6$ représente un atome d'hydrogène, un groupe phényle, un groupe phényle comportant un ou deux substituants choisis parmi un groupe alkyle en $C_1$—$C_4$, un groupe amino, un groupe hydroxyle ou un groupe alcoxy inférieur, un groupe alkyle ou un groupe alkyle en $C_1$—$C_6$ comportant un ou plusieurs substituants choisis parmi un groupe hydroxyle, un groupe amino, un groupe caboxyle ou un groupe alcoxy.

3. Procédé selon la revendication 1 ou 2, dans lequel $R^1$ représente un groupe de formule:

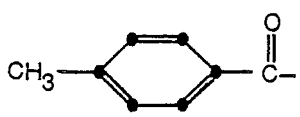 ;

b)

4. Procédé selon l'une quelconque des revendications 1 à 3, dans lequel on utilise du zinc activé.

5. Procédé selon la revendication 4, dans lequel on utilise le N,N-diméthylformamide comme solvant réactionnel.

6. Procédé de préparation d'une oxadéthiacéphalosporine à activité antibiotique, caractérisé en ce qu'il consiste à préparer un acide azétidinonesulfinique selon la revendication 1 et convertir cet acide azétidinone-sulfinique en oxadéthiacéphalosporine à activité antibiotique par des méthodes connues *en soi.*